# EUROPEAN PATENT APPLICATION

(11) **EP 3 585 136 A1**
(43) Date of publication of application: **25.12.2019**
(21) Application number: 18000541.5
(22) Date of filing: 20.06.2018
(51) Int. Cl.: H05H 1/24

(54) **A METHOD AND DEVICE FOR GENERATING LOW-TEMPERATURE ELECTRICAL WATER-BASED PLASMA AT NEAR-ATMOSPHERIC PRESSURES AND ITS USE**

(71) Applicant: Masarykova Univerzita, 602 00 BRNO 2 (CZ)
(72) Inventor: Cernák, Mirko, SK - 84107 Bratislava (SK); Krumpolec, Richard, SK - 03861 Martin (SK); Tuceková, Zlata, SK - 97901 Rimavská Sobota (SK); Kelar, Jakub, CZ - 74258 P?íbor (CZ); Zemánek, Miroslav, CZ - 67961 Letovice (CZ); Kovácik, Dusan, SK - 82106 Bratislava (SK)
(74) Representative: Danek, Vilém

(57) **Abstract**

A method and device for generating low-temperature electrical plasma stream at near-atmospheric pressures in the working gas containing more that 50 % of water vapour molecules and less that 5% of inert gas molecules, wherein the flowing working gas (1) with the temperature higher than 90 °C is fed to the discharge chamber (2), where it is ionized by the effect of a dielectric barrier discharge generating electrical plasma stream (3) with the temperature less than 500 °C using an electrode system (4) comprising at least two electrically conductive electrodes (5) situated inside of at least one dielectic body (6) with the mutual distance less than 3 mm, energized by a time alternating voltage with the frequency above 1 kHz and magnitude from 1 kV to 100 kV applied to the electrodes (5) resulting in that the ionized electrical plasma stream (3) flowing with the velocity higher than 10 cm/s in parallel and in contact with the part of dielectric body surface (7) and substantially in parallel with the electric displacement field lines is generated above of a part of the dielectric body surface (7) there exists a point in the plasma stream (3) where the electric field vector is parallel with a part of the dielectric surface above that the plasma is generated (7) and where a portion of the total flow of electric displacement field lines (8) flowing between the electrodes (5) greater than 25% is not intersecting the plasma stream (3). The device is used for surface treatment applications such as increasing surface wettability, increasing surface biocompatibility, sterilization, decontamination, hands and skin sanitization, deodorization, detoxication, etching, and for activated water generation.

## Description

### TECHNICAL FIELD

Present invention relates to a method for plasmachemical splitting of water molecules, particularly for generating stream of cold atmospheric-pressure water-based plasma with a high content of OH radicals while minimizing the generation of harmful ozone, as well as irritating nitric oxide (NO) and nitrogen dioxide (NO₂) gases. The term "atmospheric pressure" means at or near atmospheric pressure and preferably at the pressures ranging from 0.5 to 2 atm. Further, the invetion relates to a a device for generating low-temperature plasma stream at near-atmospheric pressures containing more that 50 % of water vapour molecules and less that 5 % of inert gas molecules. The device is used for surface treatment applications such as increasing surface wettability, increasing surface biocompatibility, sterilization, decontamination, hands and skin sanitization, deodorization, detoxication, etching, and for activated water generation.

### BACKGROUND OF THE INVENTION

Water dissociation by collision with high-energy electrons of electrical plasmas results in generation of many chemical species of significant practical interest.
Hydroxyl (OH) radical is an important reactive agent in various plasma applications, such as plasma sterilization, plasma medicine, and material processing. Many types of plasma sources generating large volume/area, uniformity and low temperature plasmas have been developed and used up to now to generate OH radicals by water molecules dissociation, but most of them worked at low pressures in the range of several tens Torr or less.
N. Hayashi et al.: IEEE Trans. on Plasma Sci. 36 (2008) pp.1302-1303 investigated the use of water vapour low-pressure plasma for the sterilization of medical equipment. U.S. Pat. Nos. 5,753,196 and 5,603,895 discloses other types of low-pressure water vapour plasma sources generating large volumes of diffuse water plasmas used in sterilizers for medical applications.

It is well known in the field that diffuse low-pressure water vapour plasmas are very effective also for adding OH functional groups onto surfaces. U.S. Pat. No. 5,132,108 discloses the use of low-pressure plasmas containing between 40 and 90 volume percent water vapour to enhancing the biocompatibility of polymeric surfaces. The surface modifications and cleaning by low-pressure water plasmas are decribed also in U.S. Pat. Appl. No. 20060032582, in J.H. Lee et al.: Biomaterials. 12(1991)443-8, and in Kim Shyong Siow et al.: Plasma Process. Polym. 2006, 3, 392-418. WO 2015132445 and WO 2006014591 disclose the use of low-pressure water plasmas as OH sources for Atomic Layer Depositions.

The economic and operation advantages have led to the development of a variety of plasma-based OH radical generating devices that function in humid air at 1 atm. The most widely used devices for such applications are dielectric barrier discharges (DBDs). DBDs have been known for more than a century and were originally developed for large-scale industrial ozone production. To be practicable and economic for industrial applications DBDs are typically energized by alternating voltages with frequencie ranging from 50 Hz to 13.6 MHz and magnitudes on the order of 10² to 10⁵ V.

In its simplest configuration, a DBD is a gas discharge between two parallel electrodes separated by one or more dielectric bodies, typically ceramic layers, and a gas filled gap. Therefore, this discharge requires a timevarying (e.g. oscillatory or repeatedly pulsed) voltage for its operation. When a high alternating voltage is applied to the electrodes, the electric field in the gap ionizes the gas. The ions and electrons produced are attracted towards the electrodes of opposite polarity and form a charge layer on the dielectric surface. These surface charges cancel the charge on the dilectric layer so that the electric field in the gap falls to zero, the discharge stops and the current is limited, so that the discharge does not transit to a spark or arc, and the plasma therefore remains cold. The "memory" ions created during one discharge, which are present at the dielectric surface when the next discharge occurs at the reversed voltage polarity, increase the electric field significantly and reduce the ignition and working voltage. Consequently, the memory effect of surface charges in between two successive DBD voltage phases is necessary to generate a stable, uniform and cold DBD plasmas. In the standard DBDs geometry, where the electrodes are parallel, the electric field lines have the direction perpendicular to the electrode surfaces in the whole interelectrode space, and the majority of electric displacement lines flowing between the electrodes is intersecting the surface of dielectric layer that is separating the electrodes, and above that the plasma is generated.

In ambient air DBDs break up into an array of microdischarges in the form of many thin plasma filaments. When operated with humid air, DBDs plasmas generate OH radicals, but also large amounts of harmful ozone (O₃), as well as irritating nitric oxide (NO) and nitrogen dioxide (NO₂) gases, particularly when the air contains less than 50% of water vapours. As discussed by I.G. Koo and W.M. Lee: Journal of Nuclear Sci. And Technol.42 (2005)717-723, A. Sarani et al.: Phys. Plasmas 17 (2010)063504, Z. Falkenstein and J.J. Coogan: J. Phys. D: Appl. Phys. 30 (1997) 817-825, and R. Brandenburg: Plasma Sources Sci. Technol. 26 (2017) 053001, if more water vapour is added to air, the generation of O₃, NO, and NO₂ is reduced, but the water vapour increases the ignition voltage, reduces the number of microdischarges generating high-current plasma filaments and, consequently, increases the plasma gas temperature. The increased plasma temperature is a disadvantage in many applications, for example, in treating heat-labile materials, such as cells and skin, and polymer surfaces.

U.S. Patent 5,698,164 teaches the use of an ozonizer type of dielectric barrier discharge for the atmospheric-pressure water plasma generation, even when it is apparent to those skilled in the art that the conventional DBD ozonizers are inadequate to use in high-humidity air or water vapour. The low plasma power density, limited scalability and flexibility are two main drawbacks of the atmospheric pressure plasma device for the surface treatment of hollow electrically nonconductive bodies based on the use of a surface DBD burning in water vapour described in US20160319433. F. Rehman et al. in Int. J. of Hydrogen energy 37(2012)17678-769 theoretically studied plasmolysis of water vapours at atmospheric pressure in a small DBD microchannel reactor manufactured using a combination of microfluidic and microelectronic technology.

U.S. Pat. Appl. No. US 2016/0327310 teaches a special DBD plasma source for water dissociation with microdischarges generated in microchannel plasma arrays at gas pressures up to 10 atm.

As indicated above, it is well known in the field that when a higher content water vapour is added to a plasma working gas as air or other low-cost gases, as well as in pure water steam, stable DBD discharges generating cold OH rich plasmas are hard to occur. As a solution of this problem, admixtures of expensive helium or argon in the plasma working gas are used because acceptable high power density, large area, diffuse, and low temperature water-based atmospheric-pressure plasmas efficient in OH generation are know to be achieved with these noble gases: To sterilize at 1 atm. without the toxic residue or hazardous emission U.S. Pat. Nos. 4,169,123 as well as T. Akitsu et al.: Surface & Coatings Technology 193 (2005) 29- 34 describe the DBD plasma sterilizer operating at atmospheric pressure using helium or argon diluted water plasma. T. Akitsu et al.: Surface & Coatings Technology 193 (2005) 29- 34, teach also the use of such innert gases stabilized water-based DBD plasma for the surface treatment such as improvement on surfaces of polymer materials for stronger adhesive strength. The stabilizing offect of He and Ar on DBD water-containing plasmas is described also in US 5,543,017, Chen X, Suib S L, Hayashi Y, and Matsumoto H J. Catal. 201 (2001)198, A. Sarani et al.: Physics of Plasmas 17 (2010)063504, J. Luo et al.: Res. Chem. Interned. 26(2000)849-874.

Thus, a common theme of all atmospheric pressure DBD plasma approaches to generate OH radicals is that to reach an acceptable high plasma power density and low temperatures, a minimum of 50% of an innert gas is required to prevent the appearance of undesirable hot filaments or sparks heating the plasma and reducing the efficiency of OH radical generation. Besides the costs associated with using large amounts of inert gases, it also presents an unfortunate restriction of the working range.

Since, as apparent to those skilled in the art, DBDs are inadequate to generate cold diffuse plasma in high-humidity air or water vapours, M.A. Malik and K.H. Schoenbach in J. Phys. D: Appl. Phys. 45 (2012) 132001 reviewed alternative approaches to the DBD-based atmospheric-pressure plasma sources for OH radicals generation based on the discharges, where the plasma is in direct galvanic contact with the conductive electrodes. The authors also suggested the use of a discharge gliding on a dielectric surface powered by pulsed DC voltage. K. Liu et al.: Phys. Plasmas 23 (2016) 123510 discuss in detail the problem of generating cold OH rich plasmas at atmospheric-pressures and teach the use of pulsating direct current power to excite the air-water plasma jet to reduce high plasma temperature.

Peichao Zheng et al. Applied Surface Science 259 (2012) 494-500 describe the use of DC atmospheric-pressure glow discharge generated in water vapour for the surface activation of polyimide film. However, it is difficult and tricky to reliably control such diffuse glow discharges at atmospheric pressure since minor changes in the electrode configuration or small variations of the amplitude of the applied voltage can cause a transition from the relatively unstable glow mode efficient in OH generation to that of a much more stable but less effective filamentary spark discharge. Moreover, such plasma source is not scalable to large area treatments, and the device lifetime is limited by the plasma erosion of the metallic anode due to its contact with the plasma. The similar problems are inherent to the water plasma source published by M.A. Malik and K.H. Schoenbach J. Phys. D: Appl. Phys. 45 (2012) 132001 and to the device described in I.G. Koo and W.M. Lee Jpn. J. Appl. Phys. 45(2006) 7888-7893 where water vapour, in the temperature range from 150 to 700 °C, was used as the carrier gas for DC powered electrical discharge in hollow cathode configuration.

The objective of the invention is to create technically simple method for plasmachemical splitting of water molecules, particularly for generating flux of high power density low-temperature stream of water vapour-based plasma at near atmospheric pressure without the use of stabilizing admixtures of expensive inert gases, which would eliminate the aforementioned drawbacks in generating OH radicals. Another objective of the invention is to create a device for implementing this method.

### DISCLOSURE OF THE INVENTION

The present invention seeks to provide a cost effective and technically simple method for plasmachemical splitting of water molecules, particularly for generating stream of cold atmospheric-pressure water-based plasma with a high content of OH radicals while minimizing the generation of harmful ozone, as well as irritating nitric oxide (NO) and nitrogen dioxide (NO₂) gases. The term "atmospheric pressure" means at or near atmospheric pressure and preferably at the pressures ranging from 0.5 to 2 atm. The term "water-based plasma" means the plasma gas containing more than 50 % of water molecules without little water droplets and aerosols. The term water vapour is used for air or any other gas containing water molecules.

These goals are achieved by generating a substantially diffuse plasma using an advanced type of dielectric barrier discharge with no or minimal presence of visible hot plasma filaments in a stream of water-based plasma without any significant admixture of inert gases. Using extensive experimentation, as those described in Hoder et al.: Plasma Phys. Control. Fusion 59 (2017) 074001, as well as using the results of fundamental science studies, such a those published in I.G. Koo and W.M. Lee: Journal of Nuclear Sci. And Technol.42 (2005)717-723 and M.A. Malik and K.H. Schoenbach J. Phys. D: Appl. Phys. 45 (2012) 132001, the present inventors have found that the substantionally diffuse water-based DBD plasma at the near-atmospheric pressure can be generated by inventional combination the following approaches:
(i) Employing the plasma generated in close proximity to dielectric surfaces, wherein a significant portion of the flow of electric displacement lines flowing between the electrodes, which is typically greater than 25 % is not intersecting the dielectric surface above that the plasma is generated. Consequently, there is a region in the plasma, where the component of electric field vector parallel with a part of the dielectric surface above that the plasma is generated is larger than the perpendicular component of the electric field, and generally the electric field has a significant component along the dielectric surface above that the plasma is generated. Moreover, to generate the plasma in close vicinity of a dielectric surface, the electric field. strenght must decrease with the distance from the dielectric surface above that the plasma is generated.
(ii) Generating plasma without any direct galvanic contact with any metallic electrode. This is because we found that any contact of the plasma with a metallic surface results in the formation of a hot cathode spot leading to the formation of undesired plasma hot filaments in the water-based plasma at near atmospheric gas pressures. Moreover, preventing the galvanic contact of the highly oxidative water-based plasma with the metallic electrodes eliminates the undesirable corrosion and plasma erosion of the metallic electrodes.
(iii) Keeping the plasma gas temperature higher than 90°C and less than 500 °C, since at near atmospheric pressures the water vapours do not condense in the plasma source's operating temperature at about 90 °C. Consequently, the temperature above 90°C prevents the formation of aqueous water litte droplets and aerosols, and the adsoption of water molecules on the dielectric surface, which were found to promote the undesirable plasma filamentation. Moreover, the increased plasma gas temperature reduces the plasma gas density, which is advantageous for elimination the undesired plasma hot filaments. On the other hand, it was found that temperatures approximately above 500 °C promote the plasma filamentation, apparently due to starting nonuniform electron emission from the dielectric materials used and an increased surface charge recombination, which neutralize the surface charge deposited during the previous discharge voltage pulse. As apparent to those skilled in the art, the surface charge memory effect is important in stabilizing the plasma during the subsequent discharge pulse.
(iv) Energizing the plasma by a time alternating voltage with the frequency above 1 kHz and magnitude higher than 1 kV, which generates surface charge density sufficient for starting the uniform electron emission and limits the surface charge neutralization during the period between the subsequent discharge pulses and, consequently, generates the memory effect strong enough. The term "time alternating voltage" means the voltage which causes an time alternating current in the plasma. It was found that, since the surface charge carriers do not completely decay between two subsequent voltage maxima, the voltage frequencies higher than 1 kHz result in the strong memory effect, which is reducing the temperature and increasing the homogeneity and density of the water-based plasma.
(v) Flowing the plasma working gas parallel with the dielectric surface above that the plasma is generated, and particularly as much as possible in parallel with the electric field lines.

The result of the inventional combination is a surprisingly effective method for generating stream of cold atmospheric-pressure plasma with a high content of OH radicals. The performance reflects a surprising synergy in the combination that is not suggested by or apparent from just combining the performance of either approach used alone.
The invention solves problems previously existent in the art of the DBDs applications for OH radical generation such as those presented in the background of the invention.

It was found that the use of other gases in the plasma working gas in amounts less than 50 % can increase the efficiency of OH radical generation, but still reduces significantly generation of harmful O₃, as well as irritating NO and NO₂ gases discussed in the background of the invention.

Low-temperature plasma streams generated using water vapour as a plasma gas containing more that 50 % of water molecules generated at near-atmospheric pressures according to the present invention may be useful in any OH radical rich plasma applications including the dissociation of water into hydrogen and H₂O₂ as the final desired plasmachemical products, but is specially useful for heat sensitive applications such as surface treatment, sterilization, decontamination, hands and skin sanitization, deodorization, decomposition, detoxication, etching, and activated water generation.

A preferred embodiment of the invention is the method, and the uses where pure water vapour without little water drops and earosols, also termed as water steam, is used as the plasma gas in absence of any additional gas supply system.

### BRIEF DESCRIPTION OF DRAWINGS

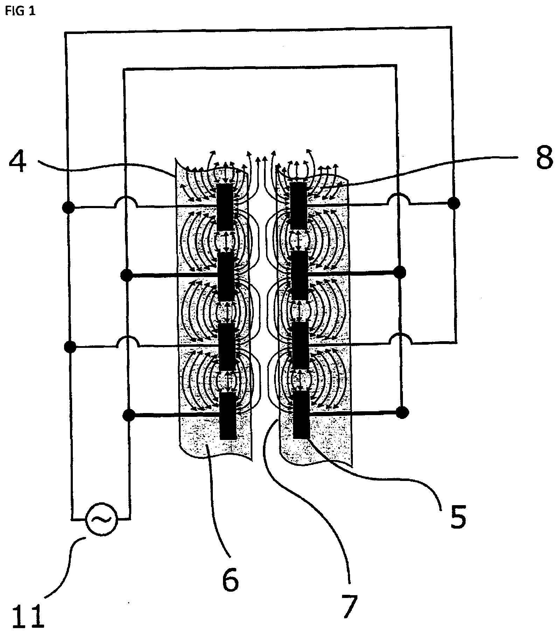

Figure 1 is a schematic cross-sectional view illustrating part of the coplanar electrode system that can be essential part of the apparatus with the electric displacement field lines flowing between the electrodes. The electrode system 4 described consists of two series of electrically conductive electrodes 5 situated inside the dielectric body 6 driven by alternating voltage power supply 11 creating characteristic electric displacement field lines 8 and generating the plasma stream in contact with dielectric body surface 7.

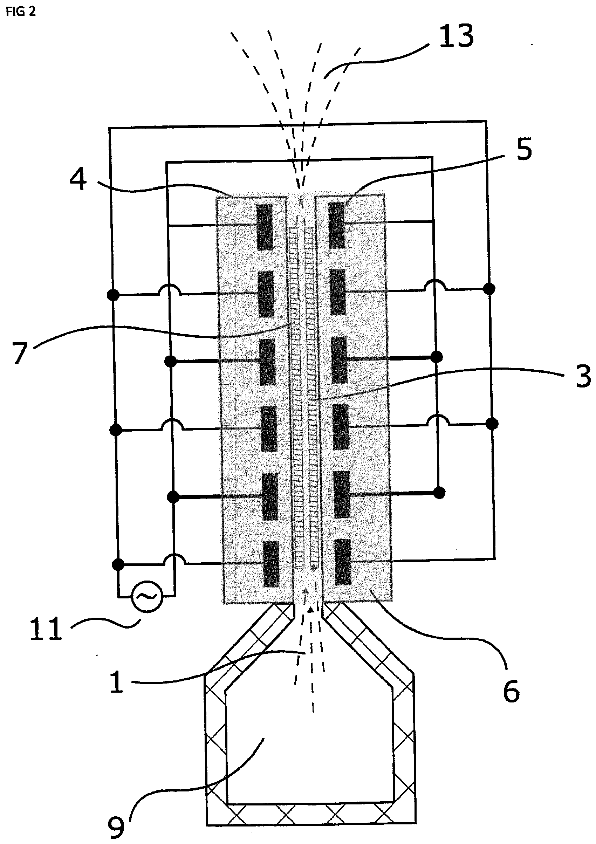

Figure 2 is a schematic cross-sectional view of the device for generating low-temperature water-based plasma stream 3 and a flow of plasma-activated water vapour 13. The device containing two coplanar electrode systems 4 is integrated with the water vapour generator 9 producing a flow of working gas 1. The electrically conductive electrodes 5 emdeded in dielectric body 6 are driven by alternating voltage power supply 11 generating water .plasma stream 3 in contact with dielectric body surface 7.

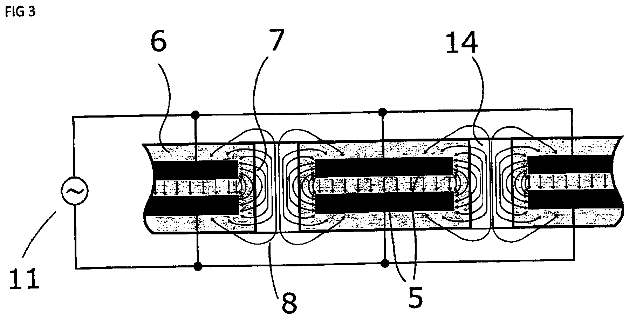

Figure 3 is a schematic cross-sectional view illustrating part of the multihollow electrode system 2 that can be essential part of the apparatus. The electrode system described consisting of system of electrically conductive electrodes 5 of two coplanar metallic planes with circular orifices 14 with diameter d embeded inside the dielectric body 6. The electrode system 5 is driven by alternating voltage power supply 11 creating electric displacement field illustrated by the electric displacement field lines 8 and generating the plasma stream in contact with dielectric body surface 7.

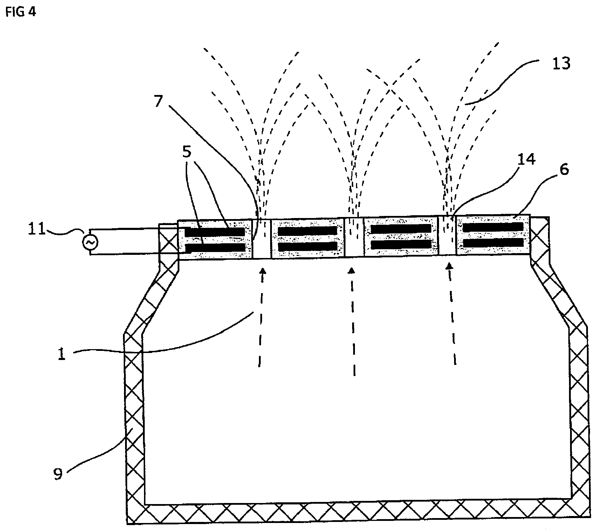

Figure 4 is a schematic cross-sectional view of the device for generating low-temperature water-based plasma stream equipped with the plasma generator containing multihollow electrode system with integrated water vapour generator 9 generating continuous flow of working gas 1. The electrode system 4 described consisting of system of electrically conductive electrodes 5 of two coplanar metallic planes with circular orifices 14 with diameter d embeded inside the dielectric body 6. The electrode system 4 is driven by alternating voltage power supply 11 and generating the plasma stream in contact with dielectric body surface 7. The device produces stream of the plasma-activated water vapour 13.

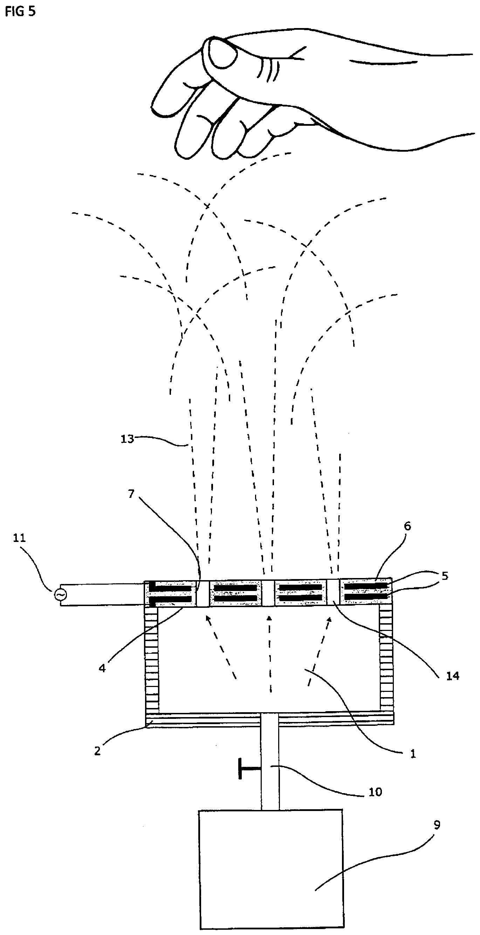

Figure 5 is a schematic cross-sectional view of the device for generating flow of low-temperature plasma-activated water vapour 13 used for sanitization of hands. The device is equipped with the plasma generator containing multihollow electrode system 4 integrated to the discharge chamber 2 and connected to the water vapour generator 9 through the flow path 10. The electrode system described is consisting of system of electrically conductive electrodes 5 of two coplanar metallic planes with circular orifices 14 with diameter d embeded inside the dielectric body 6. The electrode system 5 is driven by alternating voltage power supply 11 and generates the plasma stream in contact with dielectric body surface 7.

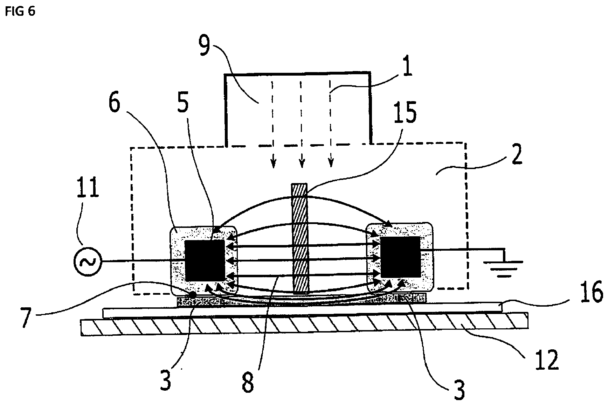

Figure 6 is a schematic cross-sectional view illustrating a tubular electrode system consisting of two dielectric bodies 6 in the form of angular dielectric tubes with inserted metallic electrodes 5. Two dielectric tubes 6 are detached by an additional dielectric barrier 15. In a close vicinity of tubular electrode system the treated planar sample 16 is moved on the dielectric surface 12 relatively to the electrode system. The discharge chamber 2 is integrated with the water vapour generator 9 feeding the discharge chamber by the flow of working gas 1. The tubular electrode system is driven by alternating voltage power supply 11 creating electric displacement field illustrated by the filed lines 8 and generating the plasma stream 3 in contact with dielectric body surface 7 and the surface of the treated sample 16.

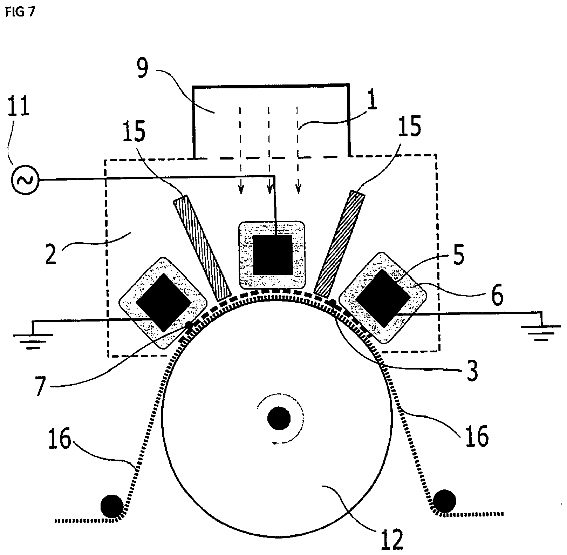

Figure 7 is a schematic cross-sectional view of illustrating tubular electrode system consisiting from angular dielectric tubes 6 with inserted metallic electrodes 5. Each two neighboring dielectric tubes are detached by a additional dielectric barrier 15. In a close vicinity of tubular electrode system a flexible sample 16 on a dielectric surface 12 is moved. Electrode system is closed in the discharge chamber 2 with the integrated generator 9 of the working gas 1. The electrode system is driven by the alternating voltage power supply 11 generating the plasma stream 3 in contact with dielectric body surface 7 and the surface of the treated sample 16.

### DETAILED DESCRIPTION

In the following, certain specific details are included and illustrated by the following non-limiting examples to provide a thorough understanding of various disclosed embodiments. Examples showing how the invention works are present also. Embodiments according to the present invention will be explained with reference to the drawings. Note that the identical elements are included in the respective embodiments described below. Therefore, the identical elements are indicated by the commom reference numerals.

### Example 1

The method according to the present invention was used for generationof low-temperature water plasma streams at atmospheric pressures. The water steam (1) superheated up to 180 °C was generated in a boiler that was used as the pure water vapour generator (9). As illustrated in Fig. 2 the water vapor generator (9) was feeding the flow of working gas (1) to the discharge chamber (2), which was constitued by the space between two electrode systems (4). Each electrode system (4) containing metal electrodes (5), 0.5 mm apart and ceramic body (6), were powered with AC voltage power source (11) generating the voltage with amplitude of 20 kV and AC current with amplitude of 500 mA arranged, as ilustrated in FIG. 2, at a distance 0.5 mm to create the discharge chamber (2). The water steam (1) was fed through discharge chamber (2) between two planar electrode systems (4) of dimensions of 8 x 20 cm at flow rate 25 L/min., where is was ionized by the effect of a dielectric barrier discharge generating electrical plasma stream (3).

The plasma stream (3) in the discharge chamber (2) was flowing substantially in parallel with the electric displacement field lines shown in Fig. 1. Also Fig. 2 illustrates that in a significant part of the discharge chamber (2), the electrical plasma stream was flowing in parallel and in contact with the part of dielectric body surfaces (7) and substantially in parallel with the electric displacement field lines. From Figs. 1 and 2 it is apparent that there exists a region in the plasma stream (3) where the electric field vector is parallel with the dielectric surfaces (7) above that the plasma is generated. Also it is apparent that a portion of the total flow of electric displacement field lines (8) flowing between the electrodes (5) greater than 25% is not intersecting the plasma stream (3).

### Example 2

The device according to the invention was embodied as a plasma surface treater in the arrangement illustrated by Figure 6.

The tubular electrode systems of the device separated by a 1.5 cm contained two dielectric bodies (6) in the form of angular alumina ceramic tubes with the wall thickness of 1 mm and the length od 20 cm with inserted copper electrodes (5). The water steam generator (9) contained an ultrasonic humidified heated up to 110 °C and generating a steady flow of water steam of 50 1/min., which was fed into the discharge chamber (2) Two dielectric tubes (6) were detached by an additional 25 cm-long alumina dielectric barrier (15) of a 0.5 mm thickness. The treated planar sample (16) was attached to and moved with the surface (12) relatively to the electrode systems with the speed less than 1 m/s in a a distance less than 1.5 mm from the tubular electrode system. The tubular electrode systems were driven by alternating voltage power supply (11) with the voltage of 50 kV magnitude and a frequency of 30 kHz. The applied voltage created the electric displacement field illustrated by the field lines (8) and generated the plasma stream (3) in contact with dielectric body surface 7 and the surface of the treated sample (16). The electric displacement field lines (8) in plasma stream (3) were preferentialy parallel to the moving direction of treated sample (16) and to the dielectric body surfaces (7) that was in contact with the plasma stream.

### Example 3

The device according to the present invention generating the stream of atmospheric-pressure wated plasma stream ilustrated by FIG. 4 contained electrode system with 27 circular orifices of multihollow dielectric barrier discharge (14) connected to the water steam generator (9). The device was equiped with electrodes (5) 0.5 mm apart and ceramic body (6) of a 1.6 mm thickness, powered with AC voltage with amplitude of 12 kV and AC current with amplitude of 500 mA (11). The generated water plasma stream (13) was fed to the open ambient air. At the distance of 2 mm from the dielectric body (6) the OH radicals with concentration of 5.10¹⁹ m⁻³ generated in plasma stream in atmosphere with more that 50 % of water vapour molecules with gas temperature of 60°C and electron temperature of 1.5 eV were measured using LIF, emission, and absorption spectroscopies.

### Example 4

According to the present invention the plasma-activated water vapour was generated using the device as described in Example 3 and was used for decontamination of PP non-woven surface. The test of plasma-activated water vapour (13) inactivation process was repeated, at distances 2 and 20 cm of the contaminated PP non-woven sample from the dielectric body (6). The planctonic as well as biofilm bacteria on polypropylene non-woven textile surface were inactivated in means of 6-3 log reduction for Escherichia coli and 3-1 log reduction for methicillin resistant Stafylococcus aureus with increasing distance of the textile. The germicidal efficiency was evaluated by standard microbiological cultivation (CFU plate counting).

### Example 6

According to the present invention the stream of water plasma was generated as described in Example 3 and was used for surface modification of polypropylene nonwovens(PET NWs) samples situated at a distances 1 mm from the device. Plasma-modified PET NWs examined increased biocompatibility measured by immobilization of biomolecules on the plasma modified PET NWs.

### Example 7

According to the present invention the stream of water plasma (3) was generated using the device and, subsequently, coolled and transferred to the plasma-activated water vapour (13) stream used for the production of plasma activated liquid water. Two planar electrode systems containing metal electrodes (5), 1 mm apart and ceramic body (6), powered with AC voltage with amplitude of 20 kV and AC current with amplitude of 500 mA (11), were arranged as ilustrated according FIG. 2, at a distance 0.5 mm to create thin discharge chamber (2) between them. The discharge chamber (2), connected to the commercial "Steam Disinfector" (9), where the water steam (1) superheated up to 180 °C is generated in a boiler, was used as the plasma-activated water vapour generator. The water steam was fed through discharge chamber at flow rate 25 L/min into the volume between two planar electrode systems (4). Generated water vapour of temperature about 95 °C was adsorbed on a cooled glass surface and trapped in a bottle. Measuring the pH of the retained water sample it was found a decrease of pH down to 3.2 from the original value 7.6 measured in the reference sample of deionized water before the plasma activation.

### Example 8

According to the present invention the method and device described in Example 1 was used for generation of the stream of the plasma-activated water vapour (13) and, subsequently the generated plasma-activated water vapour (13) stream was applied for cleaning and increasing the wettability of the surface of a planar sample (16).

The treated sample (16) was a planar steel plate moved at a distance of 5 mm from the nozzle of the discharge chamber (2)exposed to the flow of plasma-activated water vapour (13). The exposure time 0.5 s resulted in a decrease of surface contaminants and an increase of the surface wettability before finishing the surface by coat painting. The surface treated using the plasma-activated water vapour exhibited slower oxidation of treated steel surface in comparison with e.g. direct plasma cleaning by atmospheric-pressure dielectric barrier discharge.

### Example 9

The device according to the present invention was used for generation of liquid plasma activated water. Two electrode systems (4) according to the presented invention ilustrated by FIG. 1 with electrodes (5), 1 mm apart and ceramic body (6), powered with AC voltage with amplitude of 20 kV and AC current with amplitude of 500 mA (11), were arranged as ilustrated according FIG. 2, , at a distance 0.6 mm to create thin discharge chamber (2). The discharge chamber (2), connected to the commercial "Steam Disinfector" (9), where the water steam (1) superheated up to 180 °C is generated in a boiler (9), was used as the plasma-activated water vapour generator (13). The water steam was fed through discharge chamber at flow rate 30 L/min into the volume between two planar electrode systems (4) of dimensions of 8 x 20 cm. The apparatus, generated a line of plasma-activated stream of water vapour with a width of 20 cm, was used for surface treatment of planar steel plate moved at a distance of 10 mm from the nozzle of the device. The exposure time 0.5 s resulted in a decrease of surface contaminants and degreasing before surface finishing by coat painting. A method of surface cleaning using plasma-activated water vapour examined slower oxidation of treated steel surface in comparison with e.g. direct plasma cleaning by atmospheric-pressure dielectric barrier discharge.

### Example 10

According to the present invention the device described in Example 3 and illustrated by FIG.5 was used for hands and skin sanitization. The generated stream of plasma activated water vapor (13) was applied onto the surface of hands at the distance of approxinately 20 cm from the device. The device was capable to kill Escherichia coli bacteria on the hands as effectively as current sanitization methods within 3 s.

### Example 11

The device according to presented invention described in Example 2 and ilustrated by FIG.6 was used to generate water-based plasma stream for cleaning of flat float 3-mm thick float glass sample(16). The sample (16) was moved continuously in a direction parallel to the surfaces (7) at a distance 0.3 mm. A treated glass sheet (16) was situated in direct contact with the plasma stream (3). The exposure of the surface of the glass sample (16) to the plasma stream (3) led to a fast cleaning of surface contaminants, resulting an a significan increase of the glass surface wettability, where the water contact angle was decreased from 45° to 10° after 1 s of plasma treatment, to 5° after 3 s of the plasma treatment and to less than 4° after 10 s of the plasma exposure.

### Example 12

The device described in Example 4 was embodied for the treatment of flexible substrates as illustrated by FIG.7. The device illustrated by FIG.7 was used to hydrophilize the surface of a 0.2 mm thin flexible polytetrafluoroethylene (PTFE) foil sample (16). Flexible PTFE foil was guided by curved ceramic roller of curvature 1/14.8 cm⁻¹ cm (12). The static water contact angle was found to change from 118° for the untreated samples to the volue of 60° after 10 s of the plasma exposure.

### INDUSTRIAL UTILIZATION

This invention may be useful in any OH radical rich plasma applications including the dissociation of water into hydrogen and H₂O₂ as the final desired plasmachemical products, but is specially useful for heat sensitive applications such as surface treatment, sterilization, decontamination, hands and skin sanitization, deodorization, decomposition, detoxication, etching, and activated water generation.

### List of the reference symbols used

1 - flow of working gas containing more than 50 % of water steam molecules and 5 % of inert gas molecules
2 - discharge chamber
3 - electrical plasma stream
4 - electrode system
5 - electrically conductive electrodes
6 - dielectric body
7 - part of the dielectric body surface in contact with the plasma stream
8 - electric displacement field lines
9 - pure water vapour generator
10 - flow path for working gas connecting water vapour generator and discharge chamber
11 - alternating voltage power supply
12 - dielectric surface (and/or samples) moving reletively to the plasma source
13 - generated plasma-activated water vapour
14 - circular orifices of multihollow dielectric barrier discharge
15 - additional dielectric barrier
16 - sample to be treated

## Claims

1. A method for generating low-temperature electrical plasma stream at near-atmospheric pressures in the working gas containing more that 50 % of water vapour molecules and less that 5% of inert gas molecules, **characterized in that** the flowing working gas (1) with the temperature higher than 90 °C is fed to the discharge chamber (2), where it is ionized by the effect of a dielectric barrier discharge generating electrical plasma stream (3) with the temperature less than 500 °C using an electrode system (4) comprising at least two electrically conductive electrodes (5) situated inside of at least one dielectic body (6) with the mutual distance less than 3 mm, energized by a time alternating voltage with the frequency above 1 kHz and magnitude from 1 kV to 100 kV applied to the electrodes (5) resulting **in that** the ionized electrical plasma stream (3) flowing with the velocity higher than 10 cm/s in parallel and in contact with the part of dielectric body surface (7) and substantially in parallel with the electric displacement field lines is generated above of a part of the dielectric body surface (7) there exists a point in the plasma stream (3) where the electric field vector is parallel with a part of the dielectric surface (7) above that the plasma is generated and where a portion of the total flow of electric displacement field lines (8) flowing between the electrodes (5) greater than 25% is not intersecting the plasma stream (3).

2. A device for generating low-temperature plasma stream at near-atmospheric pressures containing more that 50 % of water vapour molecules and less that 5% of inert gas molecules **characterized in that** the device comprises a water vapour generator (9) operating at above atmospheric-pressure and temperature higher that 100° C, that is connected by a flow path (10) to the discharge chamber (2) containing at least two electrically conductive electrodes (5) connected to power supply (11) of a time alternating voltage with the frequency above 1 kHz and magnitude from 100 V to 100 kV, which are situated inside of at least one dielectic body (6) with the mutual distance less than 3 mm within such a position that exists a point in the plasma stream (3) where the electric field vector is parallel with a part of the dielectric surface (7) above that the plasma is generated and a portion of the total flow of electric displacement field lines (8) flowing between the electrodes (5), which is greater than 25 %, is not intersecting the plasma stream (3) generated by the device.

3. The device according to claim 2, **characterised in that** the generator of water vapour (9) containing steam boiler or ultrasound steam generator is integrated with the electrode system (4).

4. The device according to claim 2, **characterised in that** beside at least one dielectic body (6) containing at least two electrically conductive electrodes (5) connected to power supply (11) the apparatus contains at least one additional dielectric barrier (15) that is not a part of the dielectric body (6).

5. Use of the device for generating low-temperature plasma stream according to claim 2 and or 3 and or 4 in any OH radical rich plasma applications including the dissociation of water into hydrogen and H₂O₂ as the final desired plasmachemical products, and for surface treatment applications such as increasing surface wettability, increasing surface biocompatibility, sterilization, decontamination, hands and skin sanitization, deodorization, detoxication, etching, and for activated water generation.
